(19) Europäisches Patentamt
European Patent Office
Office européen des brevets

(11) Veröffentlichungsnummer: **0 405 217 A1**

(12) # EUROPÄISCHE PATENTANMELDUNG

(21) Anmeldenummer: **90111038.7**

(22) Anmeldetag: **12.06.90**

(51) Int. Cl.5: **C07D 498/04**, C07D 513/04, A61K 31/535, A61K 31/545, //(C07D498/04,265:00,205:00), (C07D513/04,279:00,205:00)

(30) Priorität: **24.06.89 DE 3920743**

(43) Veröffentlichungstag der Anmeldung: **02.01.91 Patentblatt 91/01**

(84) Benannte Vertragsstaaten: **AT BE CH DE DK ES FR GB GR IT LI LU NL SE**

(71) Anmelder: **BAYER AG**
**D-5090 Leverkusen 1 Bayerwerk(DE)**

(72) Erfinder: **Schmidt, Gunter, Dr.**
**Pahlkestrasse 63**
**D-5600 Wuppertal 1(DE)**
Erfinder: **Endermann, Rainer, Dr.**
**In den Birken 152a**
**D-5600 Wuppertal 1(DE)**
Erfinder: **Metzger, Karl Georg, Dr.**
**Pahlkestrasse 75**
**D-5600 Wuppertal 1(DE)**
Erfinder: **Haller, Ingo, Dr.**
**Dornröschenweg 4**
**D-5600 Wuppertal 1(DE)**

(54) **2-Isocepheme und 2-Oxa-isocepheme, Verfahren zu ihrer Herstellung und ihre Verwendung als und in Arzneimitteln.**

(57) Die Erfindung betrifft β-Lactamverbindungen der allgemeinen Formel (I)

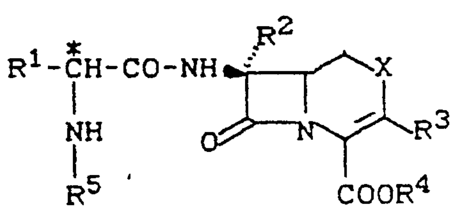

(I)

in der die Substituenten X, $R^1$, $R^2$, $R^3$, $R^4$ und $R^5$ die in der Beschreibung angegebene Bedeutung haben, Verfahren zu ihrer Herstellung sowie ihre Verwendung als Arzneimittel und in Arzneimitteln, insbesondere als antibakteriell, oral wirksame Antibiotika.

EP 0 405 217 A1

## 2-ISOCEPHEME UND 2-OXA-ISOCEPHEME, VERFAHREN ZU IHRER HERSTELLUNG UND IHRE VERWENDUNG ALS UND IN ARZNEIMITTELN

Die Erfindung betrifft β-Lactam-Antibiotika, Verfahren zu ihrer Herstellung sowie ihre Verwendung als Arzneimittel und in Arzneimitteln, insbesondere als antibakteriell, oral wirksame Antibiotika.

Es ist bekannt, daß verschiedene Vertreter von 7-α-Aminoacylcephalosporinen mit unterschiedlichen Substituenten in der 3-Stellung des Moleküls antibiotisch wirken, so z.B. Cephalexin [7-(D-α-Phenylglycylamido)-3-methyl-3-cephem-4-carbonsaure], Cefaclor [7(D-α-Phenylglycylamido)-3-chlor-3-cephem-4-carbonsäure] (vgl. GB-Patent 1 174 335; DE-OS 24 08 698 und DE-OS 27 28 578).

Weiterhin sind $C_3$-substituierte Cephalosporine als oral wirksame Verbindungen in der DE-OS 35 08 258 und in der DE-OS 35 09 618 beschrieben.

Außerdem ist bekannt,daß auch 2-Isocepheme und analoge 2-Oxa-isocephemderivate [vgl. H. Mastalerz, J. Med. Chem. 31 , 1190 (1988); EP 0 282 365; EP 0 282 895 und Farmdoc 88-126597/19] antibiotische Eigenschaften besitzen.

Die Erfindung betrifft nun β-Lactam-Verbindungen der allgemeinen Formel (I),

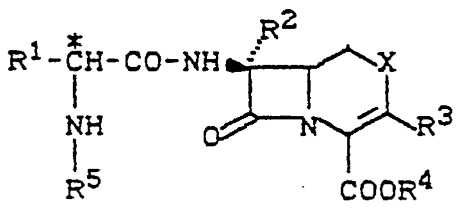

(I)

in welcher

X - für Sauerstoff oder Schwefel steht,

$R^1$ - für einen Rest der Formel

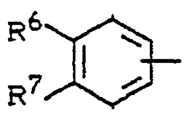

steht, worin

$R^6$ und $R^7$ gemeinsam einen 5- oder 6-gliedrigen Heterocyclus mit bis zu 3 Heteroatomen aus der Reihe Stickstoff, Schwefel und/oder Sauerstoff bilden, der gegebenenfalls durch Hydroxy, Amino, Halogen, Trifluormethyl, Cycloalkyl mit 3 bis 8 Kohlenstoffatomen, gegebenenfalls substituiertes Aryl mit 6 bis 10 Kohlenstoffatomen oder durch geradkettiges oder verzweigtes Alkyl oder Alkenyl mit jeweils bis zu 10 Kohlenstoffatomen substituiert ist, die ihrerseits durch Hydroxy, Cyano, Halogen, Amino oder Aryl mit 6 bis 10 Kohlenstoffatomen substituiert sein können,

$R^2$ - für Wasserstoff, Methoxy, oder

- für Formamido , oder

- für Hydroxylamino steht,

$R^3$ - für Wasserstoff oder

- für gegebenenfalls substituiertes, geradkettiges oder verzweigtes Alkyl mit bis zu 12 Kohlenstoffatomen steht, oder

- für Halogen, Cyano oder Azido steht, oder

- für geradkettiges oder verzweigtes Alkoxy oder Alkylthio mit jeweils bis zu 12 Kohlenstoffatomen steht, oder

- für Isopropenyl steht, oder

- für eine Gruppe der Formel $-CH=CH-R^8$ steht,

worin

$R^8$ - Wasserstoff, Halogen, Cyano, Carboxy oder Trifluormethyl bedeutet oder

- geradkettiges oder verzweigtes Alkyl, Alkoxy oder Alkoxycarbonyl mit jeweils bis zu 10 Kohlenstoffatomen oder Cycloalkyl mit 3 bis 8 Kohlenstoffatomen bedeutet oder

- gegebenenfalls substituiertes Aryl mit 6 bis 10 Kohlenstoffatomen bedeutet oder

- einen 5- bis 7-gliedrigen Heterocyclus mit bis zu 4 Heteroatomen aus der Reihe Stickstoff, Schwefel und/oder Sauerstoff bedeutet, der gegebenenfalls durch Halogen, Cyano oder durch geradkettiges oder verzweigtes Alkyl oder Alkoxy mit jeweils bis zu 8 Kohlenstoffatomen substituiert sein kann,

$R^4$ - für Wasserstoff, oder
- für eine Carboxyschutzgruppe, oder
- für einen in vivo spaltbaren Esterrest steht

und

$R^5$ - für Wasserstoff oder für eine Aminoschutzgruppe steht,

und deren pharmazeutisch verträglichen Salze.

Im Rahmen der oben angegebenen Definition steht Aryl bzw. Aralkyl im allgemeinen für einen Phenyl- oder Benzylrest, wobei die Phenylreste 1- bis 4-fach, bevorzugt 1- bis 3-fach gleich oder verschieden substituiert sein können. Als Substituenten seien zu nennen: Halogen, bevorzugt Fluor, Chlor oder Brom, geradkett ges, verzweigtes oder cyclisches Alkyl, Alkoxy, Alkylthio oder Alkylsulfonyl mit jeweils bis zu 10 Kohlenstoffatomen, bevorzugt bis zu 6 Kohlenstoffatomen, Halogenalkyl, Halogenalkoxy, Halogenalkylthio mit jeweils bis zu 7 Kohlenstoffatomen, bevorzugt mit bis zu 5 Kohlenstoffatomen und mit bis zu 5 bevorzugt bis zu 3 Chlor und/oder Fluoratomen, oder Nitro, Cyano, Benzyl, Sulfo, Amidino, Sulfamoyl, Carbamoyl oder eine gegebenenfalls substituierte Aminogruppe.

Gegebenenfalls substituiertes Alkyl im Rahmen der oben angegebenen Definition steht im allgemeinen für geradkettiges, verzweigtes oder cyclisches Alkyl mit bevorzugt bis zu 10 Kohlenstoffatomen, wobei als Substituenten in Frage kommen: Halogen, Alkoxy, Alkylthio mit jeweils bis zu 8 Kohlenstoffatomen, bevorzugt mit bis zu 6 Kohlenstoffatomen, Halogenalkylthio, Halogenalkoxy mit jeweils bis zu 8 Kohlenstoffatomen und bis zu 5, bevorzugt bis zu 3 Fluor- und/oder Chloratomen, Nitro, Cyano, eine gegebenenfalls substituierte Aminogruppe, gegebenenfalls substituiertes Aryl, Sulfo, Sulfamoyl, Alkylsulfonyl mit bis zu 6 Kohlenstoffatomen, bevorzugt mit bis zu 4 Kohlenstoffatomen, Hydroxy, Mercapto, Acyloxy, Acylthio mit jeweils bis zu 7 Kohlenstoffatomen, Carbamoyloxy, Carboxy, Alkoxycarbonyl mit bis zu 8 Kohlenstoffatomen, bevorzugt mit bis zu 6 Kohlenstoffatomen, Phenoxy, Phenylthio, Benzyloxy oder Benzylthio.

Aminoschutzgruppe im Rahmen der oben angegebenen Definition steht im allgemeinen für eine in der $\beta$-Lactam-Chemie üblichen Schutzgruppe aus der Reihe: tert.-Butoxycarbonyl, Benzyloxycarbonyl, 2-Nitrobenzyloxycarbonyl, 4-Nitrobenzyloxycarbonyl, Phenylacetyl, Allyloxycarbonyl, 2,4-Dimethoxybenzyloxycarbonyl, Allyloxymethyl, Bis-(4-methoxyphenyl)methyl, 2-(Methylthiomethoxy)ethoxycarbonyl, Trimethyl-, Triethyl-, Triphenylsilyl, tert.Butyl-dimethylsilyl, tert.-Butyl-diphenylsilyl, $C_2$-(Trimethylsilyl)ethoxy]methyl, 1-Methyl-2-benzoyl-vinyl, 1-Methyl-2-methoxy-vinyl, 1-Methyl-2-acetyl-vinyl, 1-Methyl-2-(methoxybenzoyl)-vinyl, 1-Methyl-2-(2,6-dimethoxybenzoyl )-vinyl, 1-Methyl-2-ethoxy- oder -2-methoxy-carbonyl-vinyl, Mesyl - und Ethylsulfonyl.

Der Begriff Heterocyclyl, Heterocyclyloxy oder Heterocyclylthio steht im Rahmen der oben angegebenen Bedeutung für gesättigte oder ungesättigte, gegebenenfalls über Sauerstoff oder Schwefel gebundene Heterocyclen mit bis zu 3 Stickstoffatomen, einem Sauerstoffatom und/oder einem Schwefelatom, bevorzugt für Pyrrolyl, Pyrrolidinyl, Pyrazolyl, Imidazolyl, Pyridyl, Pyridazinyl, Pyrimidyl, Pyrazinyl, Chinolyl, Isochinolyl, Indolyl, Chinoxalyl, Chinazolyl, Piperidinyl, Morpholinyl, Piperazinyl, Thiomorpholinyl, Furyl, Thienyl, Oxazolyl, Thiazolyl, Isoxazolyl, Thiadiazolyl, Triazolyl oder Tetrazolyl.

Sind diese Heterocyclen substituiert dann 1- bis 3-fach, bevorzugt 1- bis 2-fach gleich oder verschieden du geradkettiges oder verzweigtes Alkyl, Alkylthio, Alkoxy mit jeweils bis zu 4 Kohlenstoffatomen, bevorzugt mit 1 oder 2 Kohlenstoffatomen, Halogen, bevorzugt Fluor, Chlor oder Brom, Nitro, Cyano, Hydroxy, Amino, Trifluormethyl, Trifluormethoxy oder Trifluormethylthio.

Carboxyschutzgruppe im Rahmen der oben angegebenen Definition steht für die in der $\beta$-Lactam-Chemie üblichen Carboxyschutzgruppe. Bevorzugt sind leicht abspaltbare Gruppen zu nennen, wie zum Beispiel: tert.Butyl, 2,2,2-Trichlorethyl, Diphenylmethyl, Triphenylmethyl, Acetoxymethyl, Allyl, Benzyl, 4-Methoxyphenyl, 4-Nitrobenzyl, 2-Nitrobenzyl, 4-Methoxybenzyl, 2,4-Dimethoxybenzyl, Trimethylsilylethyl, Trimethylsilyl, tert.Butyl-dimethylsilyl, Acetonyl, 1-Phenoxyethyl oder 2-Methyl-2-propenyl.

Steht $R^4$ für einen in vivo leicht abspaltbaren Esterrest, so sind hiermit pharmazeutisch verträgliche Esterreste gemeint, die in vivo leicht zu freien Carboxylgruppen ($R^4$ = H) hydrolysiert werden.

Solche Esterreste sind auf dem $\beta$-Lactam-Gebiet gut bekannt. In den meisten Fällen bessern sie die Absorptionseigenschaften der $\beta$-Lactam-Verbindungen. Außerdem sollte der Rest $R^4$ von solcher Art sein, daß er einer Verbindung der Formel (I) pharmazeutisch annehmbare Eigenschaften verleiht und bei Spaltung in vivo pharmazeutisch annehmbare Fragmente freisetzt.

Beispiele für solche Gruppen befinden sich in der DE-OS 2 517 316. Bevorzugte in vivo abspaltbare Estergruppen sind die der folgenden Formeln:

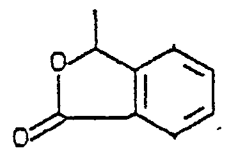 , 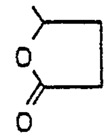 , 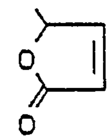 ,

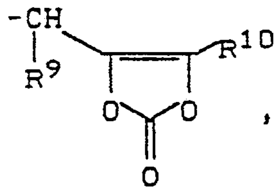 , 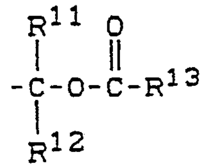 oder

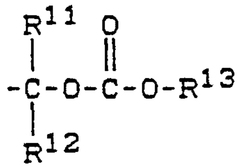

worin
$R^9$ und $R^{10}$ gleich oder verschieden sind und
- für Wasserstoff, Phenyl oder
- für $C_1$-$C_4$-Alkyl, bevorzugt für Methyl stehen,
$R^{11}$ und $R^{12}$ gleich oder verschieden sind und
- für Wasserstoff oder
- für $C_1$-$C_4$-Alkyl, bevorzugt Methyl stehen
und
$R^{13}$ - für $C_1$-$C_6$-Alkyl, bevorzugt für $C_1$-$C_4$-Alkyl steht.

Die Verbindungen der Formel I können als freie Säuren, Ester, als innere Salze oder als nicht-toxische pharmazeutisch verträgliche Salze der sauren Carboxylgruppen, wie Natrium-, Kalium-, Magnesium-, Calcium-, Aluminium-oder Ammoniumsalze, mit Aminen wie Di-, Tri-niedrigalkylaminen, Procain, Dibenzylamin, N,N'-Dibenzylethylendiamin, N-Benzyl-$\beta$-phenyl-ethylamin, N-Methyl-und N-Ethylmorpholin, 1-Ephenamin, Dihydroabietylamin, N,N'-Bis-dehydroabietylethylendiamin, N-Niedrigalkylpiperidin und anderen Aminen, die zur Bildung von Salzen von Penicillinen und Cephalosporinen verwendet werden können, vorliegen.

Wegen der Anwesenheit des mit * bezeichneten asymmetrischen Kohlenstoffatoms schließen die neuen $\beta$-Lactamantibiotika der Formel (I) die D-, L- und D,L-Form ein. Bevorzugt sind die D-Formen der erfindungsgemäßen Verbindungen der allgemeinen Formel (I).

Sowohl die Diastereomerengemische als auch die D-Form und L-Form der erfindungsgemäßen Verbindungen können zur Behandlung bakterieller Infektionskrankheiten eingesetzt werden.

Bevorzugt seien Verbindungen der allgemeinen Formel (I) genannt,
in welcher
X - für Sauerstoff oder Schwefel steht
$R^1$ - für einen Rest der Formel

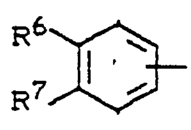

steht, worin
$R^6$ und $R^7$ gemeinsam für Pyrrolyl, Oxazolyl, Isothiazolyl, Furanyl, Pyridyl, Pyrazinyl, Pyrimidyl, Pyridazinyl, Triazolyl, Thiazolyl, Imidazolyl, Thiadiazolyl, Isoxazolyl, Thiazolinyl, Isothiazolyl, Dihydrooxazolyl oder 1,4-Dioxocyclohexenyl stehen, die gegebenenfalls durch Hydroxy, Amino, Fluor, Chlor, Brom, Trifluormethyl, Cyclopropyl, Cyclopentyl Cyclohexyl, Phenyl oder durch geradkettiges oder verzweigtes Alkyl oder Alkenyl mit jeweils bis zu 8 Kohlenstoffatomen substituiert sind, die ihrerseits durch Hydroxy, Cyano, Fluor, Chlor, Brom, Amino oder Phenyl substituiert sein können.

$R^2$ - für Wasserstoff, oder
- für Methoxy steht, oder
- für Formamido und Hydroxylamino steht,

$R^3$ - für Wasserstoff oder
- für geradkettiges oder verzweigtes Alkyl mit bis zu 8 Kohlenstoffatomen steht, das gegebenenfalls substituiert ist durch ein bis drei Fluor, Chlor, Brom, Alkoxy, Alkylthio mit jeweils bis zu 4 Kohlenstoffatomen, Halogenalkoxy, Halogenalkylthio mit jeweils bis zu 4 Kohlenstoffatomen und mit einem bis drei Fluor, Nitro, Cyano, eine gegebenenfalls substituierte Aminogruppe mit der oben angegebenen Bedeutung, Phenyl, Sulfo, Sulfamoyl, Alkylsulfonyl mit bis zu 2 Kohlenstoffatomen, Hydroxy, Mercapto, Benzyloxy, Alkanoyloxy mit bis zu 4 Kohlenstoffatomen, Carbamoyloxy, Alkoxycarbonyl mit bis zu 4 Kohlenstoffatomen, Phenyloxy, Phenylthio, Benzyloxy oder Benzylthio,
- für Fluor, Chlor, Brom, Cyano oder Azido steht,
- für geradkettiges oder verzweigtes Alkoxy oder Alkylthio mit jeweils bis zu 8 Kohlenstoffatomen steht, oder
- für Isopropenyl steht, oder
- für eine Gruppe der Formel $-CH=CH-R^8$,

worin

$R^8$ - Wasserstoff, Fluor, Chlor, Brom, Cyano, Carboxy oder Trifluormethyl bedeutet oder
- geradkettiges oder verzweigtes Alkyl, Alkoxy oder Alkoxycarbonyl mit jeweils bis zu 6 Kohlenstoffatomen, Cyclopropyl, Cyclopentyl, Cyclohexyl oder Phenyl bedeutet oder
- Pyridyl, Pyrimidyl, Furanyl oder Pyrryl bedeutet, die gegebenenfalls durch Fluor, Chlor, Brom, Cyano oder durch geradkettiges oder verzweigtes Alkyl oder Alkoxy mit jeweils bis zu 6 Kohlenstoffatomen substituiert sein können,

$R^4$ - für Wasserstoff steht, oder
- für Methyl, Ethyl, tert.Butyl, 2-Chlorethyl, 2,2,2-Trichlorethyl, Cyanoethyl, Diphenylmethyl, Triphenylmethyl, Acetoxymethyl, Allyl, Benzyl, 4-Methoxybenzyl, 2,4-Dimethoxybenzyl, 1-Phenoxyethyl, 2-Methyl-2-propenyl, 4-Nitrobenzyl, 2-Nitrobenzyl, Trimethylsilylethyl oder tert.Butyl-dimethylsilylethyl steht, oder
- für einen Rest der Formel

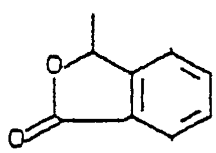 , 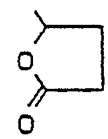 , 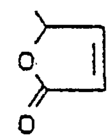 ,

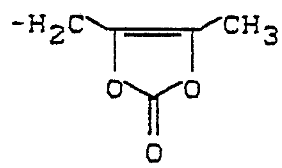 ,

$-CH_2-OCO-C(CH_3)_3$,

$-CH(CH_3)-OCOOC_2H_5$ oder

$-CH_2-OCOCH_3$ steht

und

$R^5$ - für Wasserstoff steht,

und deren pharmazeutisch verträglichen Salze.

Besonders bevorzugt seien Verbindungen der allgemeinen Formel (I) genannt, in welcher

X - für Sauerstoff oder Schwefel steht

$R^1$ - für einen Rest der Formel

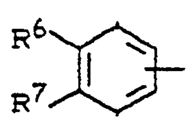

steht, worin

$R^6$ und $R^7$ gemeinsam für Pyrrolyl, Isothiazolyl, Furanyl, Oxazolyl, Pyridyl, Pyrazinyl, Triazolyl, Thiazolyl, Imidazolyl, Dihydrooxazolyl oder 1,4-Dioxocyclohexenyl stehen, die gegebenenfalls durch Hydroxy, Amino, Fluor, Chlor, Trifluormethyl, Cyclopropyl oder durch geradkettiges oder verzweigtes Alkyl mit bis zu 6 Kohlenstoffatomen substituiert sind,

$R^2$ - für Wasserstoff steht, oder

- für Methoxy steht, oder
- für Formamido und Hydroxylamino steht,

$R^3$ - für Wasserstoff oder

- für Fluor oder Chlor steht oder
- für geradkettiges oder verzweigtes Alkyl mit bis zu 6 Kohlenstoffatomen stehen, das gegebenenfalls substituiert ist durch Fluor, Chlor, Methoxy, Methylthio, Trifluormethoxy, Cyano, Phenyloxy oder Benzyloxy,
- für geradkettiges oder verzweigtes Alkoxy oder Alkylthio mit bis zu 6 Kohlenstoffatomen steht, oder
- für eine Gruppe der Formel $-CH=CH-R^8$ steht,

worin

$R^8$ - Wasserstoff, Fluor, Chlor, Cyano, Carboxy oder Trifluormethyl bedeutet oder

- geradkettiges oder verzweigtes Alkyl, Alkoxy oder Alkoxycarbonyl mit jeweils bis zu 4 Kohlenstoffatomen, Cyclopropyl, Pyridyl oder Phenyl bedeutet,

$R^4$ - für Wasserstoff steht, oder

- für einen Rest der Formel $-CH_2-OCOCH_3$,

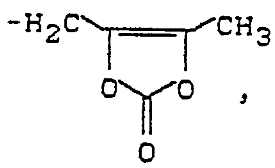 ,  $-CH(CH_3)-OCOOC_2H_5$

oder $-CH_2-OCO-C(CH_3)_3$ steht,

und

$R^5$ - für Wasserstoff steht,

und deren pharmazeutisch verträglichen Natriumsalze.

Weiterhin wurden Verfahren zur Herstellung der erfindungsgemäßen 2-Isocephem- und 2-Oxa-isocephemverbindungen, die heteroanellierte Phenylglycine in der Seitenkette enthalten, gefunden, die dadurch gekennzeichnet sind, daß man

[A] Carbonsäuren der allgemeinen Formel (II)

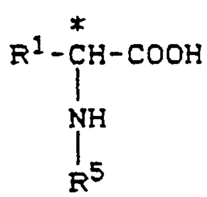

(II)

in welcher

$R^1$ und $R^5$ die oben angegebene Bedeutung haben,

nach Aktivierung der Carboxylgruppe durch Überführung in ein gemischtes Anhydrid, beispielsweise mit Chlorameisensäureethylester oder Chlorameisensäureisobutylester oder Methansulfonsäurechlorid, oder durch Überführen in das Säurehalogenid, oder durch Überführen in einen aktivierten Ester, beispielsweise mit Dicyclohexylcarbodiimid (DCC), gegebenenfalls in Anwesenheit von N-Hydroxybenzotriazol, mit den 2-Iso-cephem- bzw. 2-Oxa-isocephemgrundkörpern der allgemeinen Formel (III)

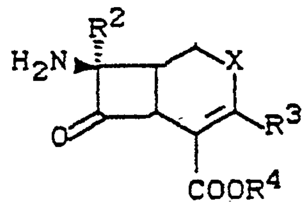 (III)

in welcher
$R^2$, $R^3$, $R^4$ und X die oben angegebene Bedeutung haben, umsetzt,
dann gegebenenfalls Schutzgruppen abspaltet und die gewünschten Salze oder aus den Salzen die freien Säuren herstellt.

Das erfindunsgemäße Verfahren kann durch das folgende Formelschema erläutert werden:

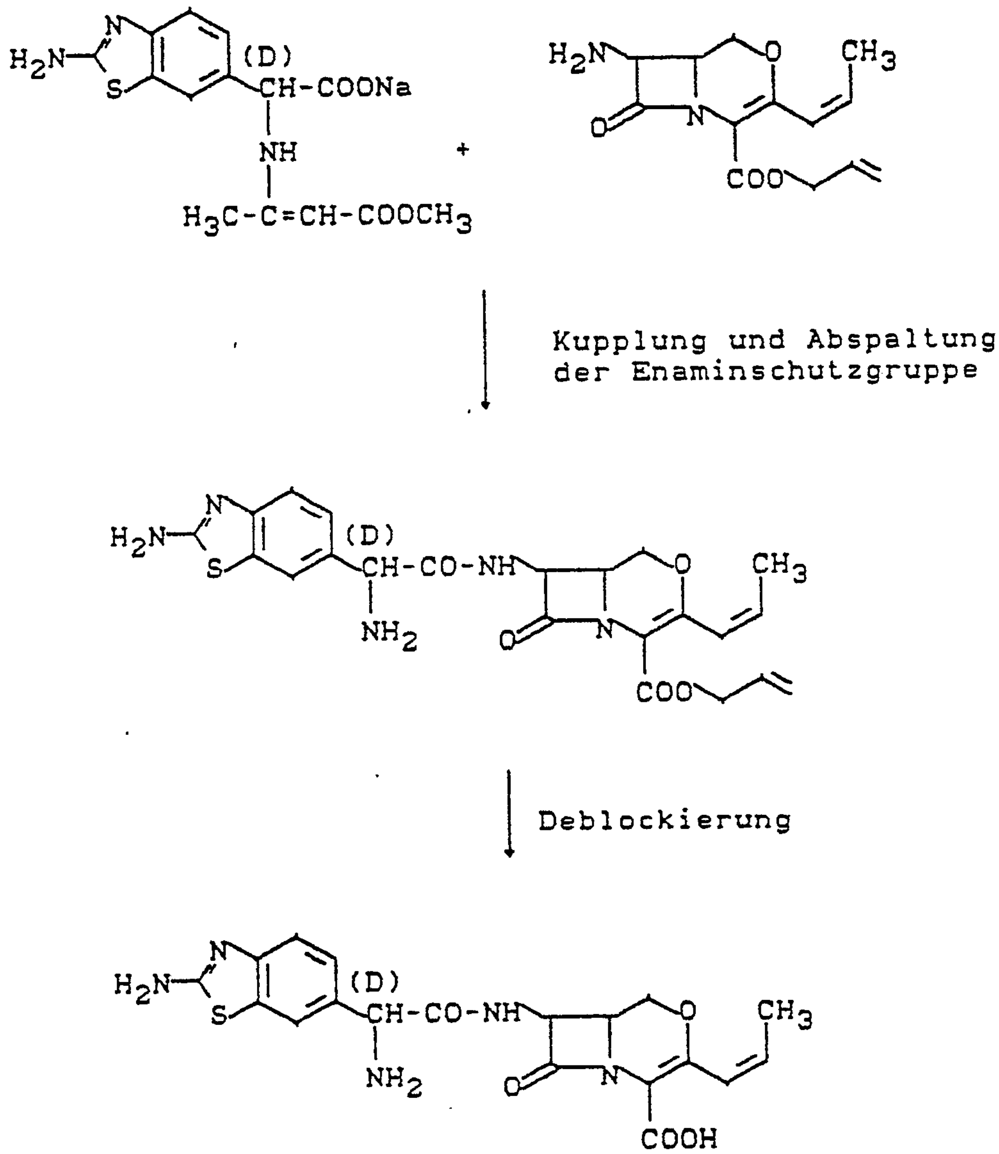


oder

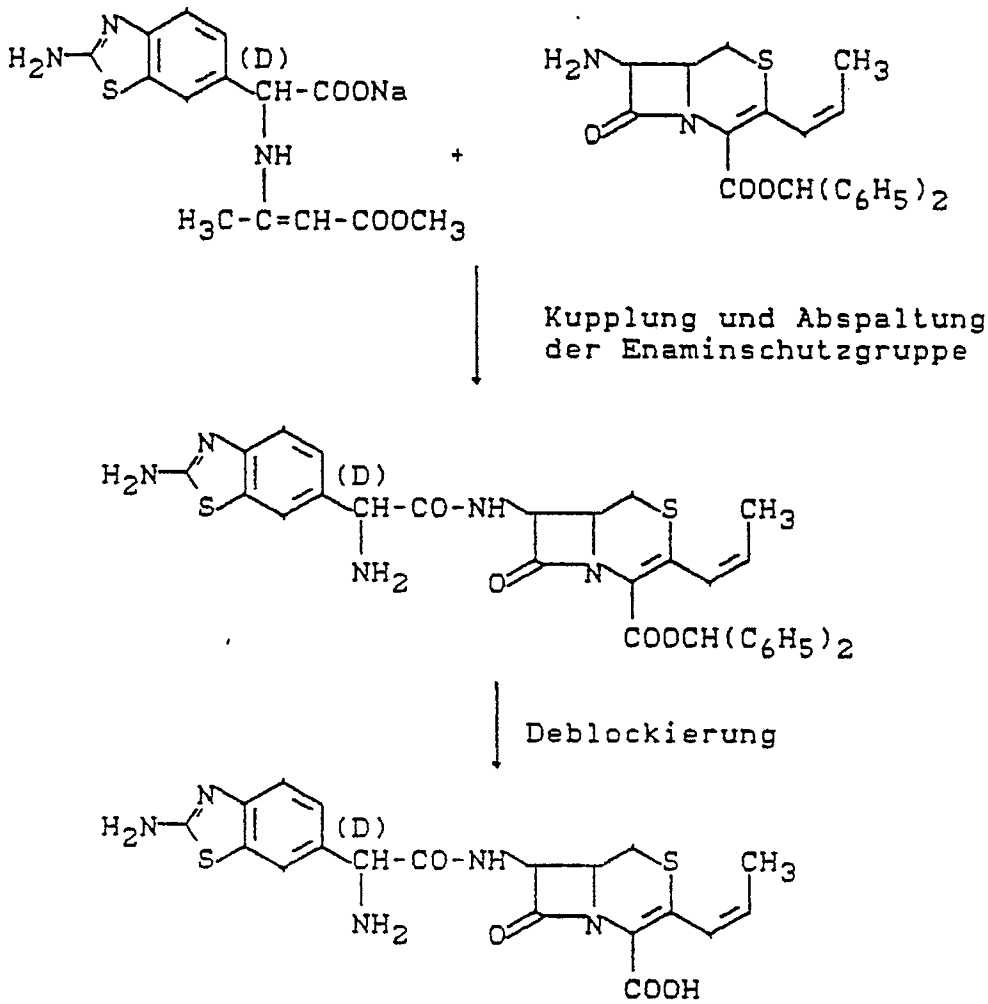


Bei der Durchführung des Verfahrens hat es sich als vorteilhaft erwiesen, die Carbonsäuren zu aktivieren und dann mit den β-Lactamaminen, die als Salze mit Amin in Lösung gebracht werden, zu kuppeln. Besonders vorteilhaft ist die Aktivierung mit Sulfonsäurederivaten der allgemeinen Formel (IV) oder mit Chlorameisensäureestern, bevorzugt Chlorameisensäureethylester, zu Anhydriden der allgemeinen Formel (Va, b), wie im folgenden Reaktionsschema verdeutlicht wird.

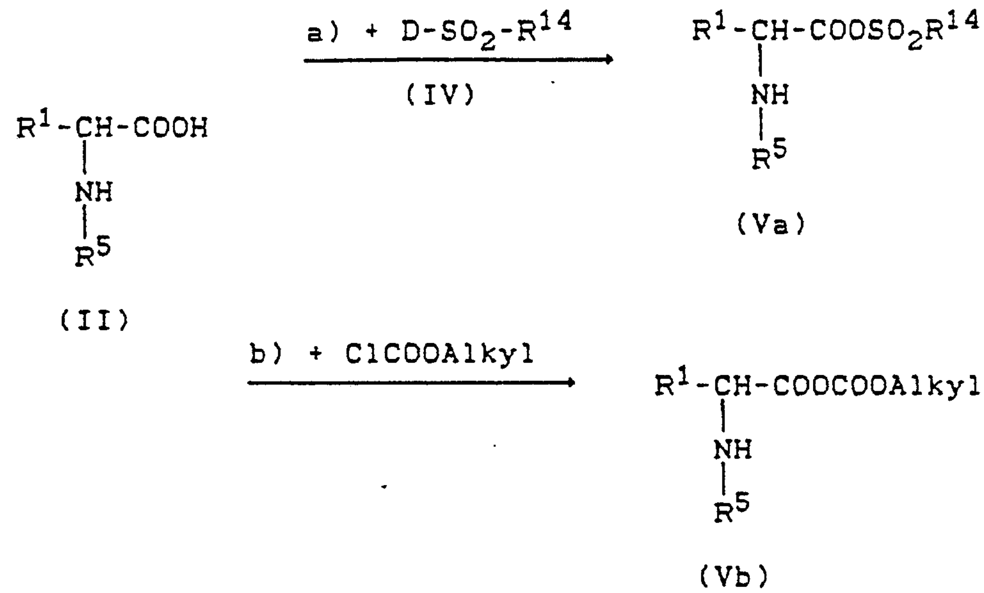


(II) (Va) (Vb)

Hierbei steht in der Formel (IV) bzw. (Va)
D - für den Rest
$R^{14}$-$SO_2$-O- oder Halogen
und
$R^{14}$- für Alkyl mit bis zu 10 Kohlenstoffatomen, das gegebenenfalls substituiert ist durch Fluor, Chlor, Cyano, Phenyl, Alkoxycarbonyl oder Alkoxy mit jeweils bis zu 4 Kohlenstoffatomen, oder
- für Phenyl, das gegebenenfalls substituiert ist durch Fluor, Chlor, Brom, Cyano, Alkyl, Alkoxy, Alkylthio, Alkoxycarbonyl oder Alkylcarbonyl mit jeweils bis zu 4 Kohlenstoffatomen, Nitro, Trifluormethyl oder Phenyl.

Wenn $R^{14}$ substituiert ist, sind bevorzugt ein bis drei Substituenten, besonders bevorzugt die oben genannten vorhanden.

Ganz besonders bevorzugt stellt $R^{14}$ einen Methyl- oder p-Tolylrest dar.

Die gemischten Anhydride der allgemeinen Formel (Va, b) werden hergestellt, indem man die Carbonsäuren der allgemeinen Formel (II) und 1- bis 1,4-Äquivalente eines Amins in einem Lösemittel löst und mit 1- bis 1,2-Äquivalenten eines Sulfonsäurederivates der Formel (IV) oder eines Chlorameisensäureesters reagieren laßt.

Als Lösemittel eignen sich alle Solventien, die sich unter den Reaktionsbedingungen nicht verändern. Hierzu gehören bevorzugt Ether wie beispielsweise Diethylether, Dioxan oder Tetrahydrofuran, oder Chlorkohlenwasserstoffe wie Methylenchlorid, Chloroform oder Tetrachlorkohlenstoff, oder Amide wie Dimethylformamid oder Hexamethylphosphorsäurtriamid, oder Acetonitril, oder Aceton. Ebenso ist es möglich, Gemische der genannten Lösemittel einzusetzen, gegebenenfalls in Mischung mit Wasser.

Als Amine eignen sich tertiäre Amine wie beispielsweise Triethylamin, Ethyldiisopropylamin oder Tributylamin, aber auch sterisch gehinderte sekundäre Amine wie beispielsweise Diisopropylamin. Ebenso können Gemische der genannten Amine eingesetzt werden.

Die Umsetzung kann bei Temperaturen zwischen -80°C und Raumtemperatur durchgeführt werden. Vorteilhaft wird die Aktivierung mit Methansulfonsäurechlorid in Dimethylformamid bei -40°C bis -60°C durchgeführt.

Zum Lösen der $\beta$-Lactamamine der allgemeinen Formel (III) können die bei der Herstellung der Verbindung der Formel (V) genannten Lösemittel oder Wasser, sowie als Base die dort genannten Amine verwendet werden.

Besonders vorteilhaft ist auch eine Aktivierung der Carbonsäure der allgemeinen Formel (II) durch Überführen in einen aktivierten Ester mit beispielsweise Dicyclohexylcarbodiimid, gegebenenfalls in Anwesenheit von N-Hydroxysuccinimid oder 1-Hydroxybenztriazol.

Als Lösemittel eignen sich hierbei alle Solventien, die sich auch für die Herstellung von Anhydriden der allgemeinen Formel (V) eignen und dort bereits aufgeführt sind. Die Umsetzungen können bei Temperaturen zwischen -30°C und +100°C durchgeführt werden. Vorteilhaft wird mit 1-Hydroxybenztriazol und Dicyclohexylcarbodiimid in Dimethylformamid bei Raumtemperatur 2 bis 6 Stunden aktiviert. Dann vom ausgefallenen Dicyclohexylharnstoff abgesaugt und mit dem $\beta$-Lactamamin der allgemeinen Formel (III) in

Form einer Lösung ihres Aminsalzes, innerhalb von 2 bis 24 Stunden umgesetzt. Zum Lösen der β-Lactamamine der allgemeinen Formel (III) können die bei der Herstellung der Verbindung der Formel (V) genannten Lösemittel sowie als Base die dort genannten Amine verwendet werden.

Die als Ausgangsverbindungen eingesetzten Carbonsäuren der allgemeinen Formel (II) sind bekannt oder können nach bekannten Methoden hergestellt werden [DE-OS 35 09 618; DE-OS 35 08 258].

Die als Ausgangsstoffe eingesetzten 2-Isocephem- bzw. 2-Oxa-isocephemgrundkörper der allgemeinen Formel (III) sind zum Teil bekannt oder können nach bekannten Methoden hergestellt werden [Farmdoc 88-126597/19; EP 0 282 895; EP 0 282 365; P. Sammes, Topics in Antibiotic Chemistry 1980 , Vol. 4, p. 89-91; J. Med. Chem. 31 , 1190 - 1196 (1988)].

Die stereochemisch einheitlichen D- bzw. L-Formen der erfindungsgemäßen Verbindungen der Formel (I) erhält man, wenn man die Diastereomerengemische durch präparative HPLC-Trennmethoden isoliert.

Andererseits erhält man die reine D- bzw. L-Form (bevorzugt die D-Form), wenn man bereits auf der Stufe der racemischen Aminosäure der Formel (II) eine chemische Racematspaltung, z.B. mit Dehydroabiethylamin, Phenylethylamin oder Camphersulfonsäure, oder eine Racematspaltung z.B. über N-Acetyl-aminosäurederivate, z.B. mit Subtilisin, Penicillinacylase oder Schweinenierenacylase, vornimmt und anschließend die stereochemisch einheitlichen D- bzw. L-Formen der Verbindungen der Formel (II) in angegebener Weise umsetzt.

Außerdem können die $C_3$-substituierten 2-Isocephemverbindungen und 2-Oxa-isocephemderivate der allgemeinen Formel (I) hergestellt werden, indem man

[B] Halogenmethyl-2-isocephem- und 2-Oxa-isocephemverbindungen der allgemeinen Formel (Ia)

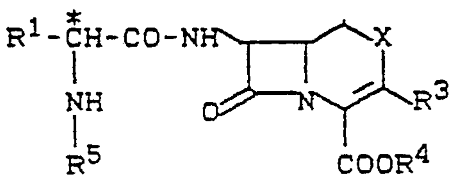

(Ia)

in welcher

$R^1$, $R^4$, $R^5$ und X die oben angegebene Bedeutung haben

und

$R^3$ - für die Gruppe der Formel $-CH_2-Cl$ steht,

in den oben erwähnten inerten Lösemitteln mit Natriumjodid, Triphenylphosphin und

$R^8-CHO$,

in welcher

$R^8$ die oben angegebene Bedeutung hat, umsetzt,

und durch Abspaltung der Schutzgruppen in die 2-Isocepheme bzw. 2-Oxa-isocepheme der allgemeinen Formel (Ib) überführt.

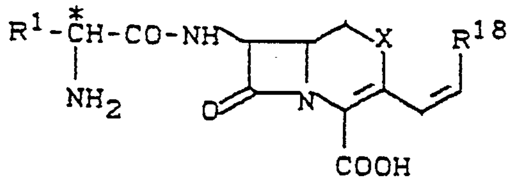

(Ib)

Die für das erfindungsgemäße Verfahren notwendigen Zwischenverbindungen können nach folgenden bekannten Methoden hergestellt werden:

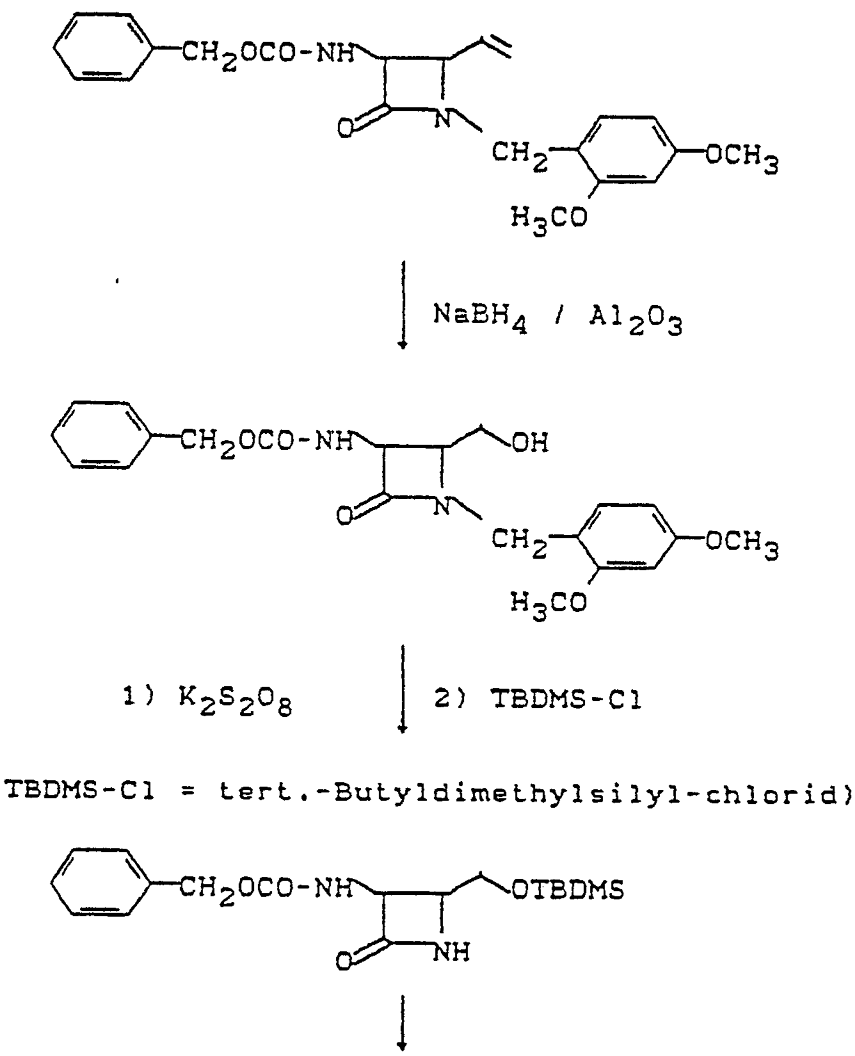

$NaBH_4$ / $Al_2O_3$
1) $K_2S_2O_8$
2) TBDMS-Cl
(TBDMS-Cl = tert.-Butyldimethylsilyl-chlorid)

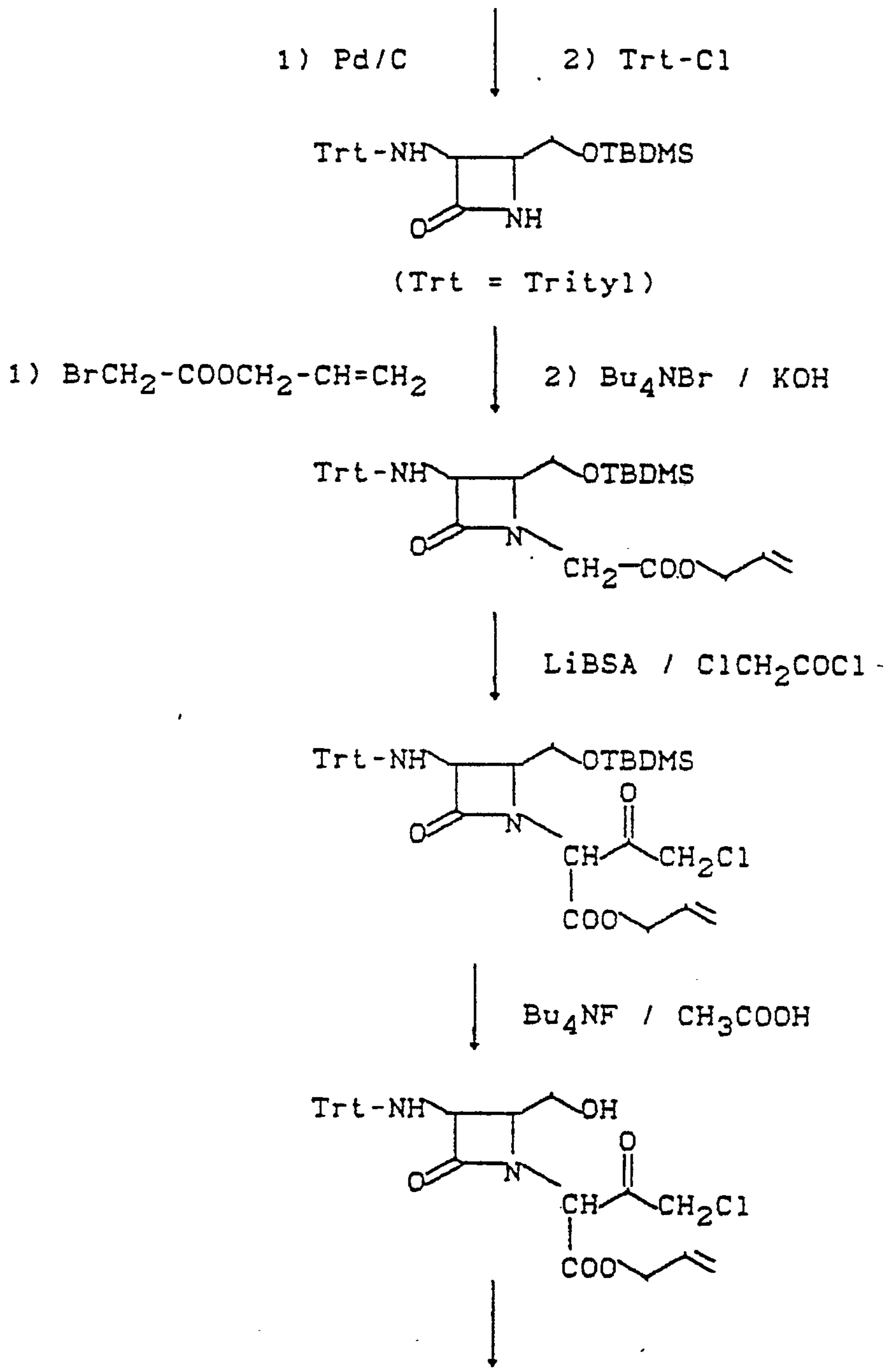

1) Pd/C
2) Trt-Cl
Trt-NH
OTBDMS
NH
(Trt = Trityl)
1) BrCH2-COOCH2-CH=CH2
2) Bu4NBr / KOH
CH2-COO
LiBSA / ClCH2COCl
CH2Cl
COO
Bu4NF / CH3COOH
OH

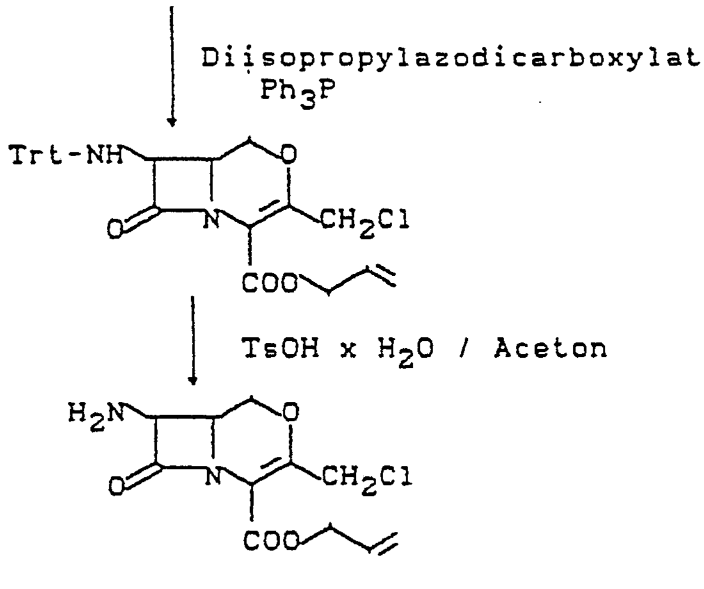


Weiterhin können $C_3$-substituierte 2-Isocephemgrundkörper bzw. 2-Oxa-isocephemnuclei der allgemeinen Formel (III) nach folgender Verfahrensvariante hergestellt werden:

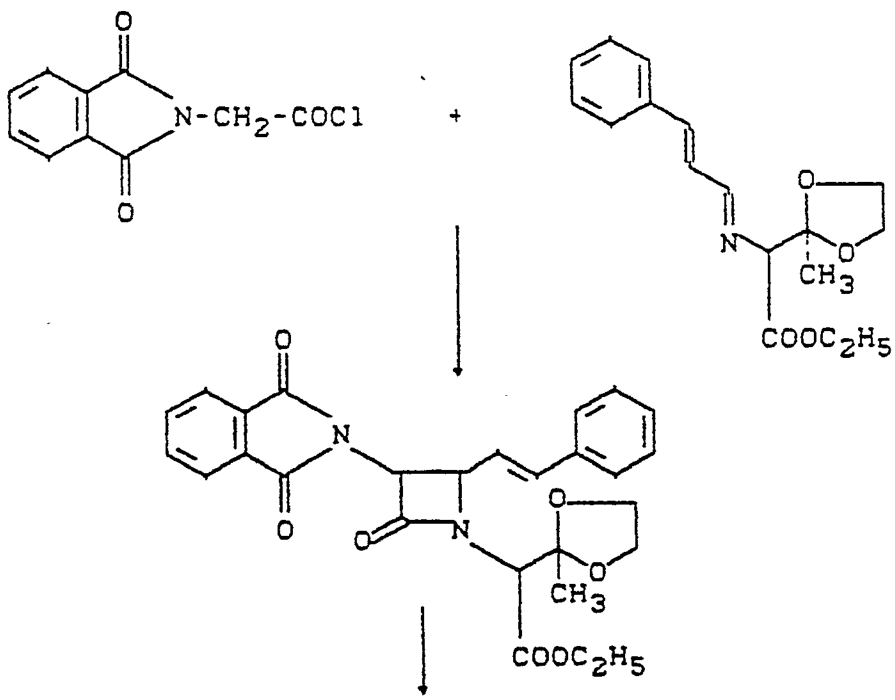

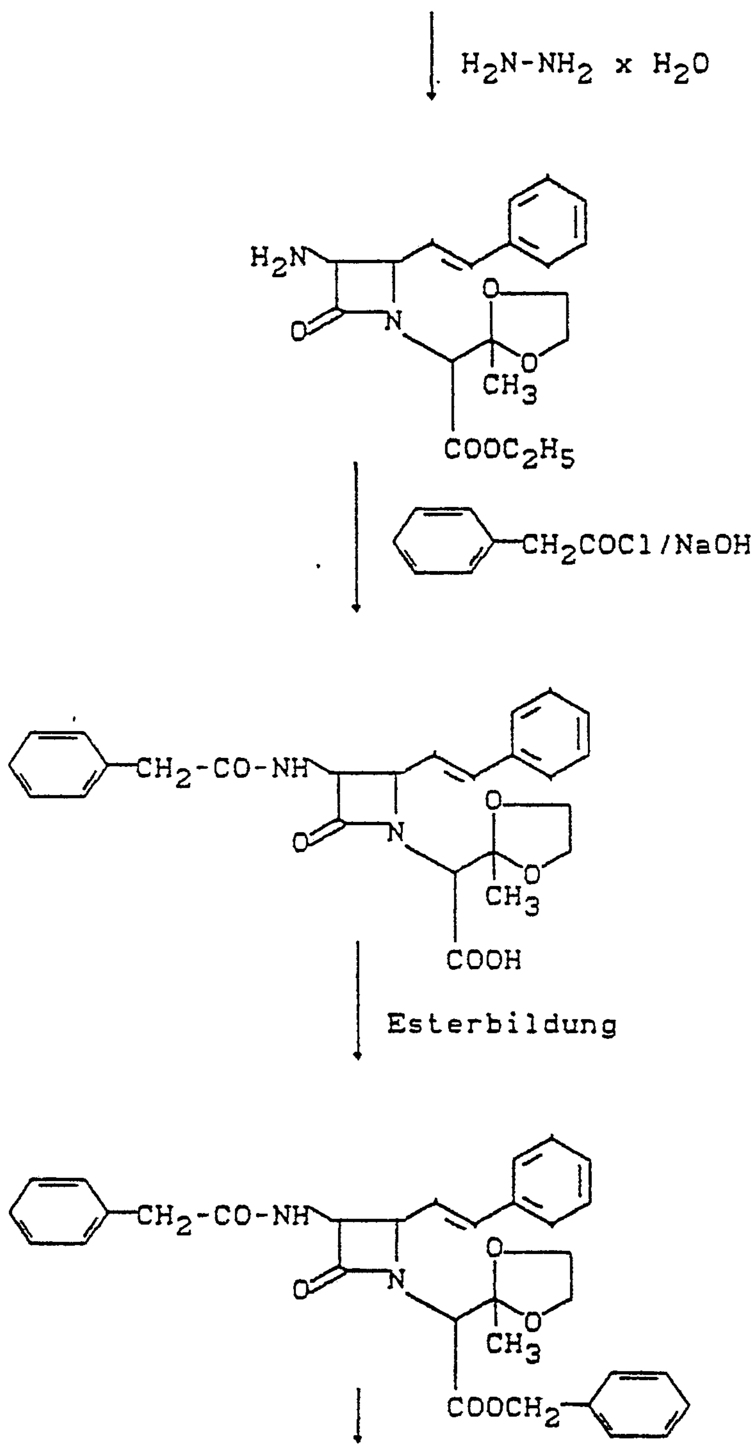
H2N-NH2 x H2O
CH2COCl/NaOH
Esterbildung

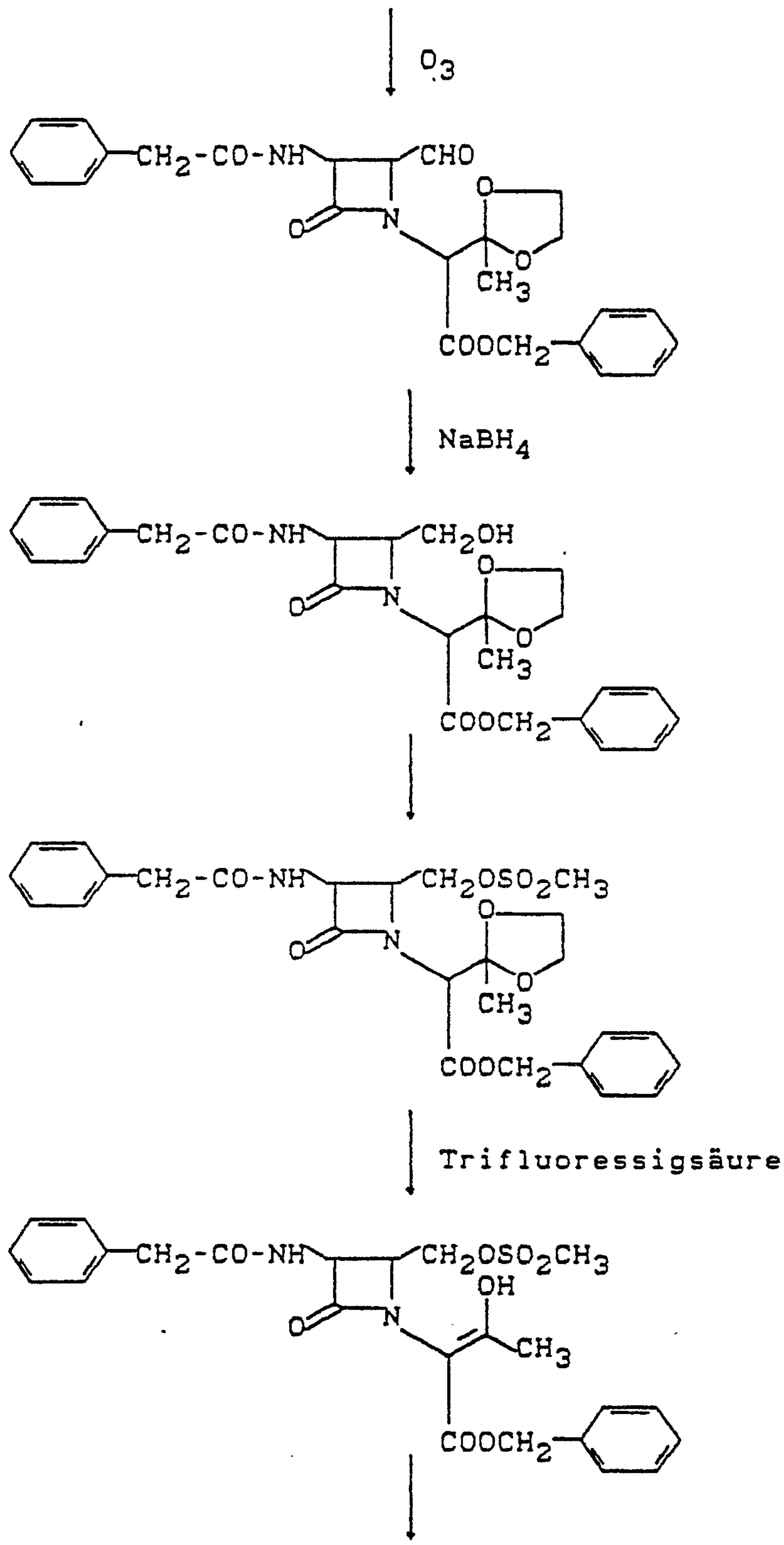
$O_3$
$NaBH_4$
Trifluoressigsäure

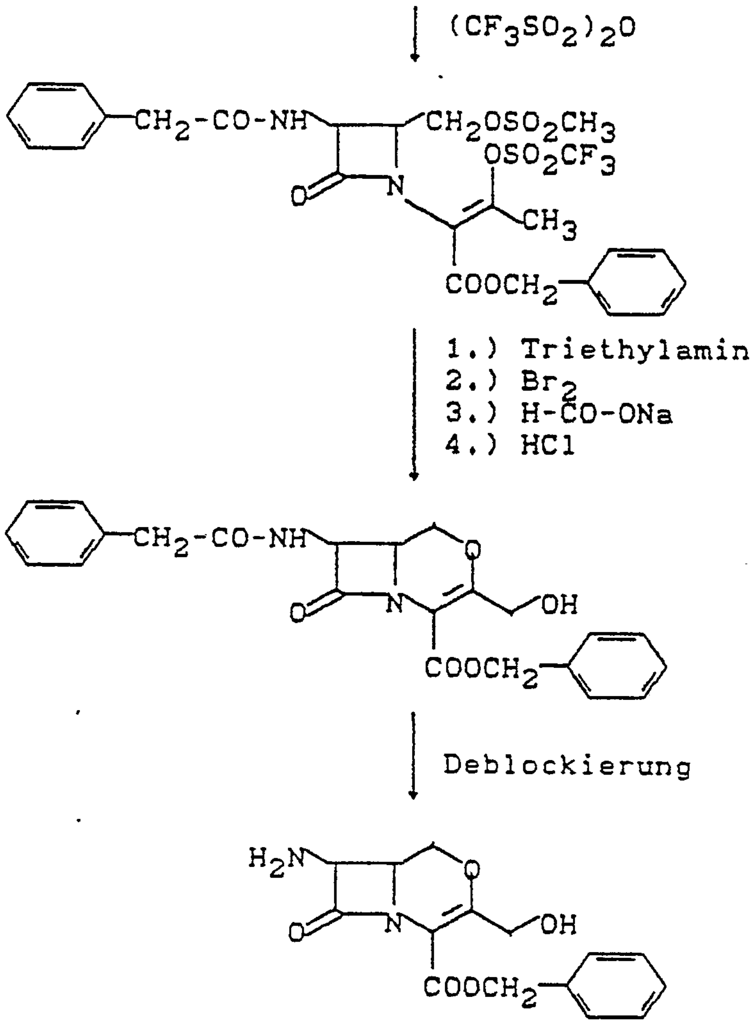


Die 2-Isocephemgrundkörper sind z.B. beschrieben in Topics in Antibiotic Chemistry 1980 , Vol. 4 von P. Sammes (J. Wiley & Sons / New York), p. 89 und Brevet francais No. 2559, 486 (13.02.1984).

Als neue erfindungsgemäße Wirkstoffe aus der Reihe der 2-Isocepheme und 2-Oxa-isocepheme seien im einzelnen, neben den experimentellen Beispielen, folgende Verbindungen genannt:

A) 2-Iso-cepheme:

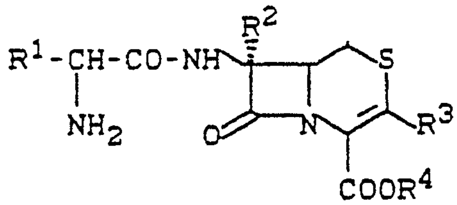

$R^2$ und $R^4$ = H

| $R^1$ | $R^3$ |
|---|---|
| 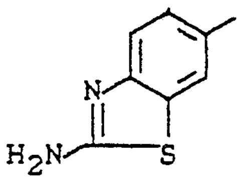 | Cl |
| 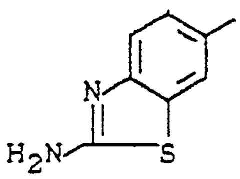 | $OCH_3$ |
| 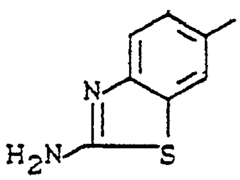 | 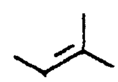 |
| 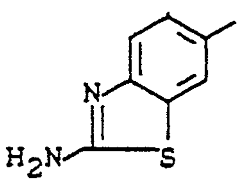 | 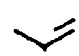 |

| $R^1$ | $R^3$ |
|---|---|
| 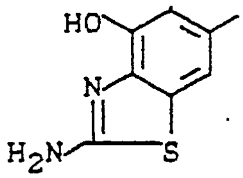 | 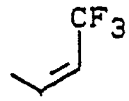 |
| 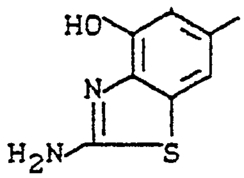 | 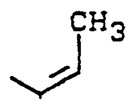 |
| 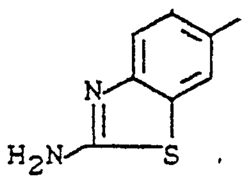 | 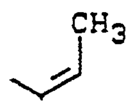 |
| 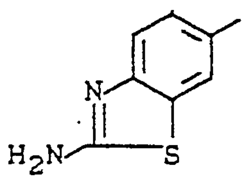 | $CH_3$ |
| 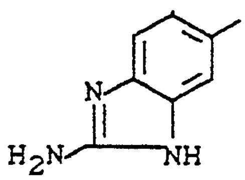 | 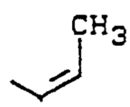 |
| 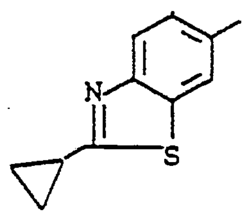 | 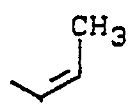 |
| 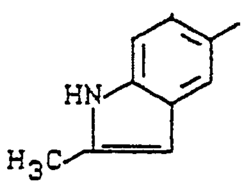 | 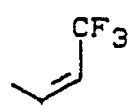 |

| $R^1$ | $R^3$ |
| --- | --- |
| 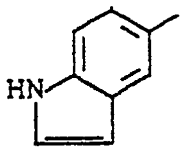 | 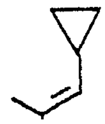 |
| 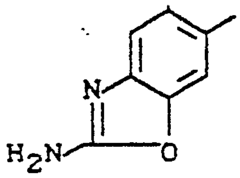 | 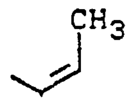 |
| 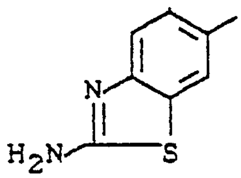 | 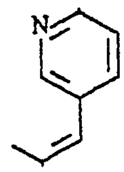 |
| 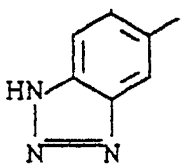 | 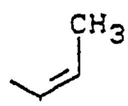 |
| 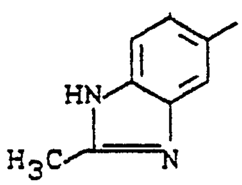 | Cl |
| 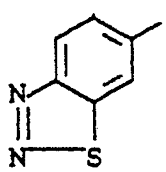 | 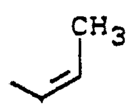 |
| 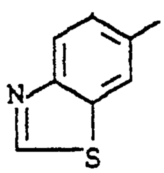 | Cl |

| $R^1$ | $R^3$ |
|---|---|
| 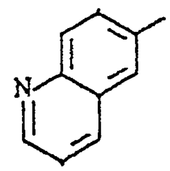 | 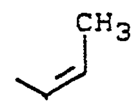 |
| 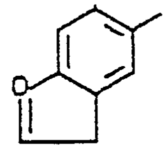 | 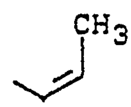 |

B) <u>2-Oxa-isocepheme:</u>

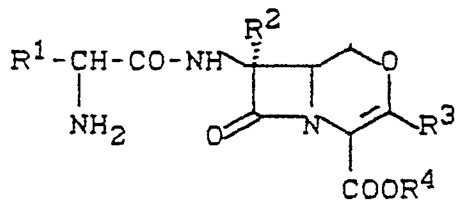

$R^2$ und $R^4$ = H

| $R^1$ | $R^3$ |
|---|---|
| 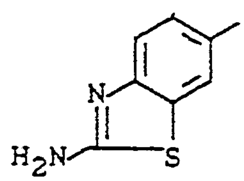 | Cl |
| 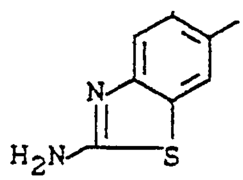 | $OCH_3$ |
| 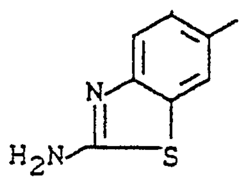 | 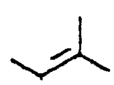 |

| $R^1$ | $R^3$ |
|---|---|
| 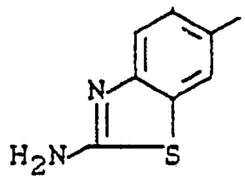 | 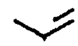 |
| 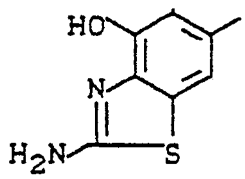 | 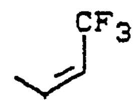 |
| 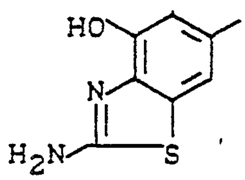 | 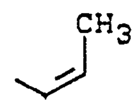 |
| 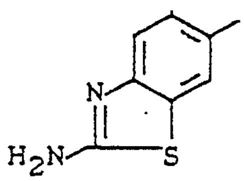 | 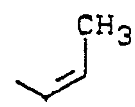 |
| 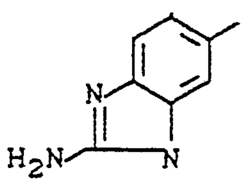 | $CH_3$ |
| 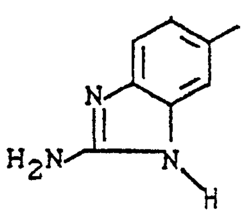 | 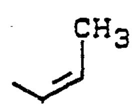 |
| 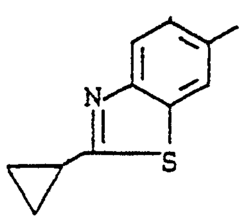 | 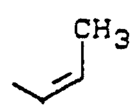 |

| $R^1$ | $R^3$ |
|---|---|
| 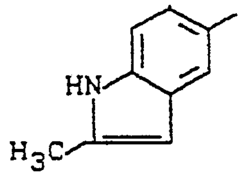 | 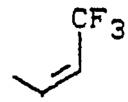 |
| 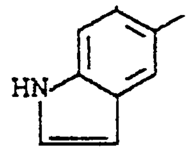 | 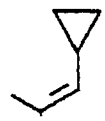 |
| 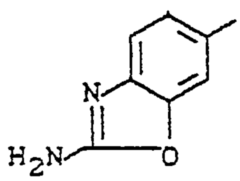 | 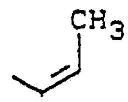 |
| 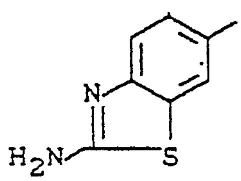 | 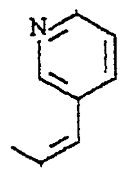 |
| 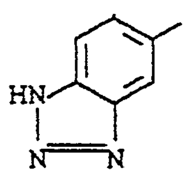 | 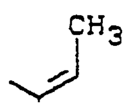 |
| 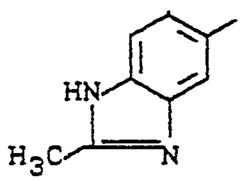 | Cl |
| 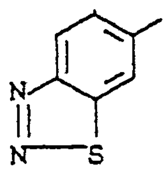 | 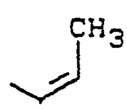 |

| $R^1$ | $R^3$ |
|---|---|
| 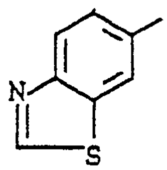 | Cl |
| 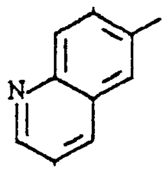 | 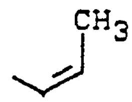 |
| 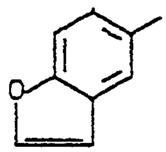 | 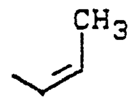 |

Als Wirkstoffe seien im einzelnen folgende Carbacephem-Derivate genannt:

A) $\Delta^3$-2-Isocephem-4-carbonsäurederivate:

D-7-[(2-Aminobenzothiazol-6-yl)-glycylamido]-1-dethia-2-thia-3-chlor-3-cephem-4-carbonsäure

D-7-[(2-Aminobenzothiazol-6-yl)-glycylamido]-1-dethia-2-thia-3-methoxy-3-cephem-4-carbonsäure

D-7-[(2-Aminobenzothiazol-6-yl)-glycylamido]-1-dethia-2-thia-3-(2-dimethyl-vinyl)-3-cephem-4-carbonsäure

D-7-[(2-Aminobenzothiazol-6-yl)-glycylamido]-1-dethia-2-thia-3-vinyl-3-cephem-4-carbonsäure

D-7-[(2-Amino-4-hydroxy-benzothiazol-6-yl)-glycylamido]-1-dethia-2-thia-3-[(Z)-3-(3,3,3-trifluoropropen-1-yl)]-3-cephem-4-carbonsäure

D-7-[(2-Amino-4-hydroxy-benzothiazol-6-yl)-glycylamido]-1-dethia-2-thia-3-[(Z)-1-propen-1-yl]-3-cephem-4-carbonsäure

D-7-[(2-Aminobenzothiazol-6-yl)-glycylamido]-1-dethia-2-thia-3-[(Z)-1-propen-1-yl]-3-cephem-4-carbonsäure

D-7-[(2-Aminobenzothiazol-6-yl)-glycylamido]-1-dethia-2-thia-3-methyl-3-cephem-4-carbonsäure

D-7-[(2-Amino-1H-benzimidazol-5(6)-yl)-glycylamido]-1-dethia-2-thia-3-[(Z)-1-propen-1-yl]-3-cephem-4-carbonsäure

D-7-[(2-Cyclopropyl-benzothiazol-6-yl)-glycylamido]-1-dethia-2-thia-3-[(Z)-1-propen-1-yl]-3-cephem-4-carbonsäure

D-7-[2-(Methyl-indol-5-yl)-glycylamido]-1-dethia-2-thia-3-[(Z)-3-(3,3,3-trifluoropropenyl)]-3-cephem-4-carbonsäure

D-7-[(Indol-5-yl)glycylamido]-1-dethia-2-thia-3-[(Z)-2-cyclopropyl-vinyl]-3-cephem-4-carbonsäure

D-7-[(2-Aminobenzoxazol-6-yl)glycylamido]-1-dethia-2-thia-3-[(Z)-1-propen-1-yl]-3-cephem-4-carbonsäure

D-7-[(2-Aminobenzothiazol-6-yl)glycylamido]-1-dethia-2-thia-3-[(Z)-2-(3-pyridyl)-vinyl]-3-cephem-4-carbonsäure

D-7-[(Benztriazol-5(6)-yl)glycylamido]-1-dethia-2-thia-3-[(Z)-1-propen-1-yl]-3-cephem-4-carbonsäure

D-7-[(2-Methyl-1H-benzimidazol-5-yl)glycylamido]-1-dethia-2-thia-3-chlor-3-cephem-4-carbonsäure

D-7-[(1,2,3-Benzothiadiazol-6-yl)glycylamido]-1-dethia-2-thia-3-[(Z)-1-propen-1-yl]-3-cephem-4-carbonsäure

D-7-[(Benzothiazol-6-yl)glycylamido]-1-dethia-2-thia-3-chlor-3-cephem-4-carbonsäure

D-7-[(Chinol-6-yl)glycylamido]-1-dethia-2-thia-3-[(Z)-1-propen-1-yl]-3-cephem-4-carbonsäure

D-7-(Benzofur-5-ylglycylamido)-dethia-2-thia-3-[(Z)-1-propen-1-yl]-3-cephem-4-carbonsäure

b) $\Delta^3$-2-Oxa-isocephem-4-carbonsäurederivate:

D-7-[(2-Aminobenzothiazol-6-yl)glycylamido]-3-chlor-$\Delta^3$-2-oxa-isocephem-4-carbonsäure

D-7-[(2-Aminobenzothiazol-6-yl)glycylamido]-3-methoxy-$\Delta^3$-2-oxa-isocephem-4-carbonsäure

D-7-[(2-Aminobenzothiazol-6-yl)glycylamido]-3-(2-dimethyl-vinyl)-$\Delta^3$-2-oxa-isocephem-4-carbonsäure

D-7-[(2-Aminobenzothiazol-6-yl)glycylamido]-3-vinyl-$\Delta^3$-2-oxa-isocephem-4-carbonsäure

D-7-[(2-Amino-4-hydroxy-benzothiazol-6-yl)glycylamido]-3-[(Z)-3-(3,3,3-trifluoropropenyl)]-$\Delta^3$-2-oxa-isocephem-4-carbonsäure

D-7-[(2-Amino-4-hydroxy-benzo-thiazol-6-yl)glycylamido]-3-[(Z)-1-propen-1-yl]-$\Delta^3$-2-oxa-isocephem-4-carbonsäure
D-7-[(2-Amino-benzothiazol-6-yl)glycylamido]-3-[(Z)-1-propen-1-yl]-$\Delta^3$-2-oxa-isocephem-4-carbonsäure
D-7-[(2-Amino-1H-benzimidazol-5(6)-yl)glycylamido]-3-methyl-$\Delta^3$-2-oxa-isocephem-4-carbonsäure
D-7-[(2-Amino-1H-benzimidazol-5(6)-yl)glycylamido]-3-[(Z)-1-propen-1-yl]-$\Delta^3$-2-oxa-isocephem-4-carbonsäure
D-7-[(2-Cyclopropyl-benzothiazol-6-yl)glycylamido]-3-[(Z)-1-propen-1-yl]-$\Delta^3$-2-oxa-isocephem-4-carbonsäure
D-7-[(2-Methyl-indol-5-yl)glycylamido]-3-[(Z)-3-(3,3,3-trifluoropropenyl)]-$\Delta^3$-2-oxa-isocephem-4-carbonsäure
D-7-[(Indol-5-yl)glycylamido]-3-[(Z)-2-cyclopropylvinyl]-$\Delta^3$-2-oxa-isocephem-4-carbonsäure
D-7-[(2-Aminobenzoxazol-6-yl)glycylamido]-3-[(Z)-1-propen-1-yl]-$\Delta^3$-2-oxa-isocephem-4-carbonsäure
D-7-[(2-Aminobenzothiazol-6-yl)glycylamido]-3-[(Z)-2-(3-pyridyl)-vinyl]-$\Delta^3$-2-oxa-isocephem-4-carbonsäure
D-7-[(Benztriazol-5(6)-yl)glycylamido]-3-[(Z)-1-propen-1-yl]-$\Delta^3$-2-oxa-isocephem-4-carbonsäure
D-7-[(2-Methyl-1H-benzimidazol-5-yl)glycylamido]-3-chlor-$\Delta^3$-2-oxa-isocephem-4-carbonsäure
D-7-[(1,2,3-Benzothiadiazol-6-yl)glycylamido]-3-1-propen-1-yl]-$\Delta^3$-2-oxa-isocephem-4-carbonsäure
D-7-[(Benzothiazol-6-yl)glycylamido]-3-chlor-$\Delta^3$-2-oxa-isocephem-4-carbonsäure
D-7-[(Chinol-6-yl)glycylamido]-3-[(Z)-1-propen-1-yl]-$\Delta^3$-2-oxa-isocephem-4-carbonsäure
D-7-(Benzofur-5-ylglycylamido)-3-[(Z)-1-propen-1-yl]-$\Delta^3$-2-oxa-isocephem-4-carbonsäure.

Die erfindunsgemäßen Verbindungen der allgemeinen Formel I weisen bei geringer Toxizität ein breites antibakterielles Spektrum gegen gram-positive und gram-negative Keime auf. Diese Eigenschaften ermöglichen ihre Verwendung als chemotherapeutische Wirkstoffe in der Human-und Tiermedizin.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:
Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae Citrobacter (Citrob. freundii, Citrob. divernis), Salmonella und Shigella; ferner Klebsiellen (Klebs. pneumoniae, Klebs oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tüberculosis. Insbesondere wirken die erfindungsgemäßen Stoffe gegen Staphylokokken, Streptokken,Enterokokken und Haemophilus influenzae. Bei parenteraler oder insbesondere oraler Verabreichung sind die neuen Verbindungen gegen Mikroorganismen wie Staphylokokken, Streptokokken, Enterobakteriaceen, Escherichia coli, Klebsiella, Salmonella, Shigella und Proteus sehr gut wirksam.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:

Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen, der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, post-operative Wundinfektionen, Abszesse, Phlegmone, Wundinfektionen, infizierte Verbrennungen, Brandwunden, In fektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Menigitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:

Schwein: Coli-diarrhoe, Enterotoxämie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonie, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutiche Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetyl-alkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Starke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten,

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische

dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erf indungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. Lactamaseinhibitoren z.B. mit Penicillinen, die besonders penicillinasefest sind und Clavulansäure kombiniert werden. Eine solche Kombination wäre z.B. die mit Oxacillin oder Dicloxacillin.

Die erfindungsgemäßen Verbindungen können zum Zweck der Erweiterung des Wirkungsspektrums und um eine Wirkungssteigerung zu erreichen( auch mit Aminoglykosidantibiotica, wie z.B. Gentamicin, Sisomicin, Kanamicin, Amikacin oder Tobramicin kombiniert werden.

## Herstellungsbeispiele

### Beispiel 1

D-7-[(2-Aminobenzothiazol-6-yl)glycylamido]-3-[(Z)-1-propen-1-yl]-$\Delta^3$-2-oxa-isocephem-4-carbonsäure

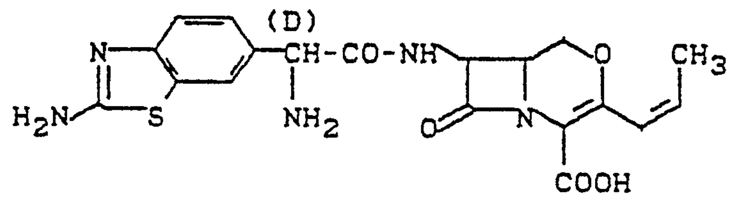

A) D-7-[N-(Allyloxycarbonylamino)-2-(2-aminobenzothiazol-6-yl)glycylamido]-3-chlormethyl-$\Delta^3$-2-oxa-isocephem-4-carbonsäure-allylester

Zu einer Mischung von D-N-[(Allyloxy)carbonyl]-2-(2-aminobenzothiazol-6-yl)glycin und p-Toluolsulfonsäuresalz von 7-Amino-3-chlormethyl-$\Delta^3$-2-oxa-isocephem-4-carbonsäureallylester in Tetrahydrofuran und Dimethylformamid in Gegenwart von Triethylamin gibt man N,N'-Dicyclohexylcarbodiimid (DCC) gelöst in Tetrahydrofuran bei 20 °C hinzu, rührt anschließend 3,5 Stunden bei Raumtemperatur und filtriert von ausgefallenem Dicyclohexylharnstoff ab. Das Filtrat wird im Vakuum zur Trockene eingeengt, der Rückstand in Essigester aufgelöst, zweimal mit gesättigter Natriumhydrogencarbonat-Lösung und danach mit wäßriger Kochsalzlösung gewaschen. Nach dem Trocknen über Magnesiumsulfat wird das Lösemittel abdestilliert, wobei ein öliger Rückstand hinterbleibt.

B) D-7-[N-(Allyloxycarbonylamino)-2-(2-aminobenzothiazol-6-yl)glycylamido]-3-[(Z)-1-propen-1-yl]-$\Delta^3$-2-oxa-isocephem-4-carbonsäure-allylester

Eine Mischung der Verbindung aus Beispiel 1(A), Natriumiodid und Triphenylphosphin werden in Aceton 5 Stunden bei Raumtemperatur gerührt. Nach Zugabe von Ether wird das ausgefallene Phosphoniumsalz abgesaugt und in einem Gemisch von Dichlormethan und Methanol in Anwesenheit von Natriumcarbonat in Wasser bei 0 bis 5 °C gelöst. Nun erfolgt die Zugabe von Acetaldehyd, wobei die Temperatur bis 26 °C ansteigen kann Man rührt 2,5 -3 Stunden bei Raumtemperatur. Anschließend gibt man Dichlormethan und Wasser hinzu, trennt die organische Schicht ab, filtriert die Dichlormethan-Phase über Kieselgel 60 (0,04 -0,063 mm), wäscht solange mit Methylenchlorid bis die Lösung farblos wird. Das Methylenchlorid-Eluat wird im Vakuum bis zur Trockene eingeengt, der Rückstand in Toluol aufgenommen und auf eine Säule, die mit Kieselgel (0,04 - 0,063 mm) gepackt ist, gegeben. Zunächst wird mit Toluol und danach mit dem Lösemittelgemisch Toluol/Essigester (5:1) und Toluol/Essigester (1:1) eluiert.

C) D-7-[(2-Aminobenzothiazol-6-yl)glycylamido]-3-[(Z)-1-propen-1-yl]-$\Delta^3$-2-oxa-isocephem-4-carbonsäure

Die Verbindung aus Beispiel 1(B) wird in Methylenchlorid gelöst und unter Argon mit Tetrakis-(triphenylphosphin)-palladium (0) und Triphenylphosphin behandelt. Danach gibt man unter Rühren N-Methylanilin hinzu und rührt 60 Minuten. Das ausgefallene Produkt wird abgesaugt, mit Dichlormethan gewaschen und im Vakuum getrocknet.

Das Rohprodukt wird in Wasser suspendiert, mit 2 N Salzsäure bei pH 1,2 in Lösung gebracht, über ein Kieselgur-Bett filtriert und mit 0,1 N Salzsäure nachgewaschen. Das Filtrat wird auf eine RP 18-Säule (Hibar 250 - 25, 7 µm, Merck) gepumpt, danach zunächst mit Wasser und anschließend mit 5%igem Methanol und zum Schluß mit 10%igem Methanol eluiert. Die Fraktionen, die das Z-isomere Derivat enthalten, werden vereinigt, von Methanol befreit und die zurückbleibende wäßrige Lösung lyophilisiert.

In Analogie zur Herstellungsvorschrift des Beispiels 1 können aus dem literaturbekannten Zwischenprodukt 7-Amino-3-chlormethyl-$\Delta^3$-2-oxa-isocephem-4-carbonsäureester folgende Beispiele hergestellt werden.

Beispiel 2

D-7-[(Indol-5-yl)glycylamido]-3-[(Z)-1-propen-1-yl]-$\Delta^3$-2-oxa-isocephem-4-carbonsaure

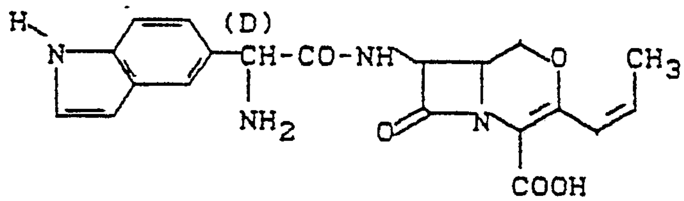

Beispiel 3

D-7-[(2-Aminobenzoxazol-6-yl)glycylamido]-3-[(Z)-1-propen-1-yl]-$\Delta^3$-2-oxa-isocephem-4-carbonsäure

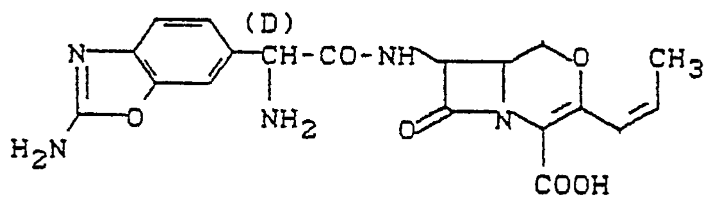

**Ansprüche**

1. $\beta$-Lactam-Verbindungen der allgemeinen Formel (I),

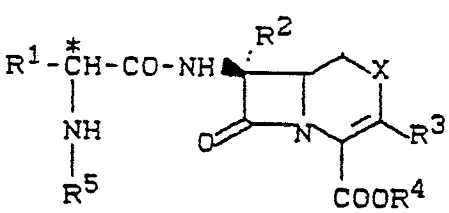

(I)

in welcher
X - für Sauerstoff oder Schwefel steht,
$R^1$ - für einen Rest der Formel

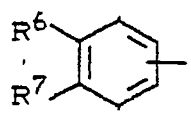

steht, worin
$R^6$ und $R^7$ gemeinsam einen 5- oder 6-gliedrigen Heterocyclus mit bis zu 3 Heteroatomen aus der Reihe Stickstoff, Schwefel und/oder Sauerstoff bilden, der gegebenenfalls durch Hydroxy, Amino, Halogen, Trifluormethyl, Cycloalkyl mit 3 bis 8 Kohlenstoffatomen, gegebenenfalls substituiertes Aryl mit 6 bis 10 Kohlenstoffatomen oder durch geradkettiges oder verzweigtes Alkyl oder Alkenyl mit jeweils bis zu 10 Kohlenstoffatomen substituiert ist, die ihrerseits durch Hydroxy, Cyano, Halogen, Amino oder Aryl mit 6 bis 10 Kohlenstoffatomen substituiert sein können,
$R^2$ - für Wasserstoff, Methoxy, oder
- für Formamido , oder
- für Hydroxylamino steht,
$R^3$ - für Wasserstoff oder
- für gegebenenfalls substituiertes, geradkettiges oder verzweigtes Alkyl mit bis zu 12 Kohlenstoffatomen

steht, oder
- für Halogen, Cyano oder Azido steht, oder
- für geradkettiges oder verzweigtes Alkoxy oder Alkylthio mit jeweils bis zu 12 Kohlenstoffatomen steht, oder
- für Isopropenyl steht, oder
- für eine Gruppe der Formel -CH=CH-$R^8$ steht, worin
$R^8$ - Wasserstoff, Halogen, Cyano, Carboxy Trifluormethyl bedeutet oder
- geradkettiges oder verzweigtes Alkyl, Alkoxy oder Alkoxycarbonyl mit jeweils bis zu 10 Kohlenstoffatomen oder Cycloalkyl mit 3 bis 8 Kohlenstoffatomen bedeutet oder
- gegebenenfalls substituiertes Aryl mit 6 bis 10 Kohlenstoffatomen bedeutet oder
- einen 5- bis 7-gliedrigen Heterocyclus mit bis zu 4 Heteroatomen aus der Reihe Stickstoff, Schwefel und/oder Sauerstoff bedeutet, der gegebenenfalls durch Halogen, Cyano oder durch geradkettiges oder verzweigtes Alkyl oder Alkoxy mit jeweils bis zu 8 Kohlenstoffatomen substituiert sein kann,
$R^4$ - für Wasserstoff, oder
- für eine Carboxyschutzgruppe, oder
- für einen in vivo spaltbaren Esterrest steht
und
$R^5$ - für Wasserstoff oder für eine Aminoschutzgruppe steht,
und deren pharmazeutisch verträgliche Salze.

2. Verbindungen nach Anspruch 1 zur Anwendung in einem Verfahren zur therapeutischen Behandlung des menschlichen oder tierischen Körpers.

3. Verfahren zur Herstellung der Verbindungen der Formel I gemäß Anspruch 1, dadurch gekennzeichnet, daß man
[A] Carbonsäuren der allgemeinen Formel (II)

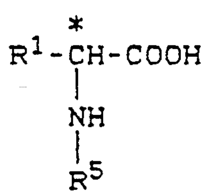

(II)

in welcher
$R^1$ und $R^5$ die in Anspruch 1 angegebene Bedeutung haben,
nach Aktivierung der Carboxylgruppe durch Überführung in ein gemischtes Anhydrid, oder durch Überführen in das Säurehalogenid oder durch Überführen in einen aktivierten Ester, gegebenenfalls in Anwesenheit von N-Hydroxybenztriazol,
mit den 2-Iso-cephem- bzw. 2-Oxa-isocephemgrundkörpern der allgemeinen Formel (III)

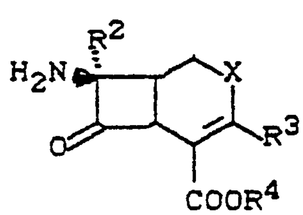

(III)

in welcher
$R^2$, $R^3$, $R^4$ und X die in Anspruch 1 angegebene Bedeutung haben, umsetzt,
dann gegebenenfalls Schutzgruppen abspaltet und die gewünschten Salze oder aus den Salzen die freien Säuren herstellt.

4. Verfahren zur Herstellung der Verbindungen der Formel 1 gemäß Anspruch 1, dadurch gekennzeichnet, daß
[B] Halogenmethyl-2-isocephem- und 2-0xa-isocephemverbindungen der allgemeinen Formel (la)

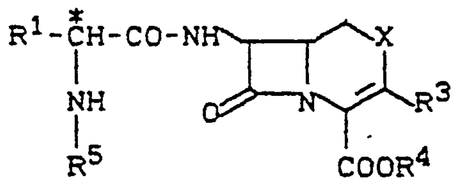

(Ia)

in welcher

$R^1$, $R^4$, $R^5$ und X die in Anspruch 1 angegebene Bedeutung haben

und

$R^3$ - für die Gruppe der Formel $-CH_2-Cl$ steht,

in inerten Lösemitteln mit Natriumjodid, Triphenylphosphin und

$R^8$-CHO,

in welcher

$R^8$ die in Anspruch 1 angegebene Bedeutung hat, umsetzt,

und durch Abspaltung der Schutzgruppen in die 2-Isocepheme bzw. 2-Oxa-isocepheme der allgemeinen Formel (Ib) überführt.

5. Arzneimittel, enthaltend Verbindungen der Formel I gemäß Anspruch 1.

6. Verwendung von Verbindungen der Formel I gemäß Anspruch 1 zur Herstellung von Arzneimitteln.

Europäisches Patentamt

# EUROPÄISCHER RECHERCHENBERICHT

Nummer der Anmeldung

EP 90 11 1038

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-313081 (OTSUKA PHARMACEUTICAL CO.,LTD.)<br>* Seite 3, Zeile 20 - Seite 4, Zeile 51 *<br>--- | 1, 5 | C07D498/04<br>C07D513/04<br>A61K31/535<br>A61K31/545<br>//(C07D498/04, 265:00,205:00)<br>(C07D513/04, 279:00,205:00) |
| A,D | EP-A-282365 (ROUSSEL-UCLAF)<br>* Seiten 2 - 3, Zeile 62; Ansprüche 1, 7 *<br>--- | 1, 5 | |
| A | EP-A-187456 (ICI PHARMA)<br>* Seite 1 *<br>* Seite 5, Zeile 19 - Seite 5, Zeile 28 *<br>--- | 1, 5 | |
| A,D | Journal of Medicinal Chemistry<br>vol. 31, no. 6, 1988, Columbus,Ohio<br>Seiten 1190 - 1196; Harold Mastalerz et al.:<br>"An Examination of O-2-Isocephems as Orally Absorbable Antibiotics"<br>* das ganze Dokument *<br>----- | 1, 5 | |

RECHERCHIERTE SACHGEBIETE (Int. Cl.5 )

C07D498/00
C07D513/00

Der vorliegende Recherchenbericht wurde für alle Patentansprüche erstellt

| Recherchenort | Abschlußdatum der Recherche | Prüfer |
|---|---|---|
| BERLIN | 28 SEPTEMBER 1990 | KYRIAKAKOU, G. |

2

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument